# FASCICULE DE BREVET EUROPEEN

(11) **EP 3 490 517 B1**
(45) Date de publication et mention de la délivrance du brevet: **27.05.2020**
(21) Numéro de dépôt: 17742786.1
(22) Date de dépôt: 28.07.2017
(51) Int. Cl.: A61H 3/00

(54) **STRUCTURE D'EXOSQUELETTE POUR L'ASSISTANCE A L'EFFORT D'UN UTILISATEUR**
EXOSKELETTSTRUKTUR ZUR BEREITSTELLUNG VON KRAFTUNTERSTÜTZUNG FÜR EINEN BENUTZER
EXOSKELETON STRUCTURE THAT PROVIDES FORCE ASSISTANCE TO THE USER

(30) Priorité: 28.07.2016 FR 1657295
(43) Date de publication de la demande: 05.06.2019
(73) Titulaire: Safran Electronics & Defense, 92100 Boulogne-Billancourt (FR); B-Temia Inc., St-Augustin-de-Desmaures, QC G3A 2J9 (CA)
(72) Inventeur: SOUCY, Francisco, G3A 2J9 Québec (CA); BILODEAU, Katia, G3A 2J9 Québec (CA); ZOSO, Nathaniel, G3A 2J9 Québec (CA); BAPTISTA, Jonathan, 92100 Boulogne-Billancourt (FR); GRENIER, Jordane, 92100 Boulogne-Billancourt (FR)
(74) Mandataire: Regimbeau
(86) Numéro de dépôt international: PCT/EP2017/069228
(87) Numéro de publication internationale: WO 2018/020032

(56) Documents cités:
- EP-A2- 0 361 405
- EP-A2- 2 923 686
- WO-A1-97/38759
- WO-A1-2012/002078
- US-A- 3 826 251
- US-A- 5 230 696
- US-A1- 2004 102 723
- US-A1- 2007 161 934

## Description

### DOMAINE DE L'INVENTION

L'invention concerne un sous-ensemble d'exosquelette pour l'assistance à l'effort d'un utilisateur.

### ETAT DE LA TECHNIQUE

Les exosquelettes d'assistance à l'effort sont des structures mécaniques positionnées en parallèle du squelette humain et qui permettent d'améliorer les capacités physiques du corps humain.

Il existe différents types d'exosquelettes, dont la forme et la structure dépendent des tâches à accomplir par l'utilisateur. Les deux principaux types d'exosquelettes sont ceux destinés à l'assistance aux mouvements de l'utilisateur d'une part, et ceux destinés à la démultiplication des capacités de force de l'utilisateur d'autre part.

Dans le cas des exosquelettes destinés à l'assistance aux mouvements de l'utilisateur, l'utilisateur doit généralement transporter la structure de l'exosquelette puisque celle-ci est disposée sur son corps, ce qui a pour conséquence de limiter la liberté de mouvement de l'utilisateur et de générer une charge supplémentaire et de la fatigue associée.

Afin de soulager l'utilisateur, on connait des structures d'exosquelette dans lesquelles une partie de la masse de l'exosquelette est transférée au sol via des plaques disposées sous les pieds de l'utilisateur et reliées au reste de la structure.

Dans ces structures, les pieds de l'utilisateur ne sont pas en contact avec le sol, ce qui rend la structure peu confortable.

De plus, du fait de la présence des plaques, la mobilité de l'utilisateur est nécessairement réduite. En effet, afin d'assurer un transfert de la masse de l'exosquelette vers le sol, ces structures n'autorisent généralement pas pleinement la rotation ou la prono-supination de la cheville de l'utilisateur.

Cela a pour conséquence que ce type de structure ne permet pas de procurer un appui sur le sol dans toutes les phases de marche et/ou sur tous types de sols, notamment lorsque l'utilisateur marche sur un sol en pente ou irrégulier.

Le document US 5 230 695 décrit un joint pivotant pour une attelle de genou comprenant une tige fémorale et une tige tibiale, reliées entre elles par une charnière. La charnière comprend une fente incurvée fixée à l'une des tiges et un pion fixé à l'autre tige et coulissant à l'intérieur de la fente. Le joint pivotant comprend également une bague en caoutchouc autorisant ou s'opposant, selon la position du pion, au coulissement du pion dans la fente dans le sens d'hyperextension du genou.

### RESUME DE L'INVENTION

Un but de l'invention est de proposer une solution pour soulager l'utilisateur des charges qu'il porte, que ce soit la charge générée par la structure de l'exosquelette en elle-même, par des éléments externes pouvant être associés à la structure de l'exosquelette (par exemple un sac à dos) ou le poids même de l'utilisateur, tout en présentant un meilleur confort et une meilleure mobilité.

Ce but est atteint dans le cadre de la présente invention grâce à un sous-ensemble d'exosquelette comprenant :
- une première pièce d'exosquelette,
- une deuxième pièce d'exosquelette,
- un ensemble de liaison reliant la première pièce d'exosquelette à la deuxième pièce d'exosquelette, l'ensemble de liaison comprenant un guide monté fixe par rapport à l'une de la première pièce et de la deuxième pièce, et un pion monté fixe par rapport à l'autre de la première pièce et de la deuxième pièce, le pion étant monté coulissant à l'intérieur du guide entre une première position d'extrémité et une deuxième position d'extrémité.

L'ensemble de liaison comprend en outre un dispositif de limitation agencé pour autoriser une rotation du pion par rapport au guide lorsque le pion est dans la première position d'extrémité, et s'opposer à la rotation du pion par rapport au guide lorsque le pion est dans la deuxième position d'extrémité.

Le dispositif de limitation comprend un élément élastique avec lequel la première pièce d'exosquelette vient en prise lorsque le pion est dans la deuxième position d'extrémité, l'élément élastique exerçant sur la première pièce d'exosquelette une force de rappel élastique tendant à s'opposer à une rotation relative entre la première pièce d'exosquelette et la deuxième pièce d'exosquelette aussi bien dans un premier sens de rotation que dans un deuxième sens de rotation, opposé au premier sens de rotation.

Un tel sous-ensemble d'exosquelette peut être utilisé de sorte que :
- lorsque le sous-ensemble n'est pas chargé, le pion se trouve dans la première position d'extrémité, le dispositif de limitation autorisant une rotation relative entre la première pièce d'exosquelette et la deuxième pièce d'exosquelette, permettant ainsi une liberté de mouvement entre les deux pièces,
- lorsque le sous-ensemble est chargé, le pion se trouve dans la deuxième position d'extrémité, le dispositif de limitation s'opposant à une rotation relative entre la première pièce d'exosquelette et la deuxième pièce d'exosquelette, permettant ainsi un transfert d'effort entre la première pièce et la deuxième pièce.

Lorsque le pion est dans la deuxième position, le dispositif de limitation s'oppose à une rotation relative entre la première pièce et la deuxième pièce via la pièce élastique. De ce fait, le dispositif de limitation autorise une certaine rotation entre la première pièce et la deuxième pièce, tout en générant une force de rappel s'opposant à ce mouvement afin d'assurer le transfert d'effort entre la première pièce et la deuxième pièce. Cette caractéristique procure à l'utilisateur un meilleur confort lors de ses mouvements.

L'une de la première pièce d'exosquelette et de la deuxième pièce d'exosquelette est par exemple une pièce propre à être fixée à une jambe de l'utilisateur et l'autre de la première pièce d'exosquelette et de la deuxième pièce d'exosquelette est une pièce propre à être fixée au pied de l'utilisateur.

L'ensemble de liaison entre les deux pièces d'exosquelette est alors placé en parallèle de l'articulation de la cheville de l'utilisateur.

Pendant le cycle de marche, le pion est déplacé alternativement de la première position à la deuxième position (lorsque l'utilisateur pose le pied au sol : chargement) et de la deuxième position à la première position (lorsque l'utilisateur lève le pied du sol : déchargement).

Lorsque le pion se trouve dans la première position d'extrémité (pied levé), l'ensemble de liaison autorise une rotation de la deuxième pièce par rapport à la première pièce causée par un mouvement du pied par rapport à la jambe de l'utilisateur.

Lorsque le pion se trouve dans la deuxième position d'extrémité (pied en appui sur le sol), l'ensemble de liaison s'oppose à la rotation de la deuxième pièce par rapport à la première pièce, de manière à transférer la charge supportée par l'exosquelette vers le sol et à supporter tout ou partie du couple exercé sur la cheville de l'utilisateur.

Dans un premier mode de réalisation de l'invention, l'ensemble de liaison est disposé entre la première pièce d'exosquelette et la deuxième pièce d'exosquelette de sorte que, lorsque le pion se trouve dans la première position d'extrémité, l'ensemble de liaison autorise une rotation de la deuxième pièce d'exosquelette par rapport à la première pièce d'exosquelette causée par un mouvement de flexion/extension du pied par rapport à la jambe.

Dans un deuxième mode de réalisation, l'ensemble de liaison est disposé entre la première pièce d'exosquelette et la deuxième pièce d'exosquelette de sorte que, lorsque le pion se trouve dans la première position d'extrémité, l'ensemble de liaison autorise une rotation de la deuxième pièce d'exosquelette par rapport à la première pièce d'exosquelette causée par un mouvement d'éversion/inversion du pied par rapport à la jambe.

Le sous-ensemble d'exosquelette peut en outre présenter les caractéristiques suivantes :
- le guide comprend une lumière oblongue ménagée dans la première pièce d'exosquelette,
- le pion présente une forme cylindrique de révolution,
- l'élément élastique est disposé entre les deux pièces d'exosquelette,
- l'élément élastique est monté fixe par rapport à la deuxième pièce d'exosquelette,
- l'élément élastique est un bloc en matériau élastique,
- la première pièce d'exosquelette présente une saillie, et l'élément élastique présente un renfoncement dans lequel la saillie est reçue lorsque le pion est dans la deuxième position d'extrémité,
- la saillie présente une forme complémentaire de la forme du renfoncement,
- la saillie présente une forme générale de pointe et le renfoncement présente une forme générale en V,
- la première pièce d'exosquelette présente un évidement, et l'élément élastique présente un renflement propre à être reçu dans l'évidement lorsque le pion est dans la deuxième position d'extrémité,
- l'élément élastique présente une ou plusieurs portion(s) propres à être comprimées entre les deux pièces d'exosquelette lorsque le pion est dans la deuxième position d'extrémité, en cas de rotation relative entre la première pièce d'exosquelette et la deuxième pièce d'exosquelette,
- l'élément élastique comprend un ressort agencé pour exercer une force de rappel sur l'autre pièce d'exosquelette, la force de rappel exercée par le ressort s'opposant à la rotation du pion par rapport au guide lorsque le pion est dans la deuxième position d'extrémité,
- le ressort comprend une ou plusieurs lame(s) flexible(s), chaque lame ayant une extrémité fixée à l'une des deux pièces d'exosquelette, la ou les lame(s) étant disposée(s) de sorte que la rotation du pion par rapport au guide a pour effet que l'autre pièce d'exosquelette sollicite la ou les lame(s) en flexion.

L'invention se rapporte en outre à une structure d'exosquelette comprenant un sous-ensemble tel que défini précédemment.

### PRESENTATION DES DESSINS

D'autres caractéristiques et avantages ressortiront encore de la description qui suit, laquelle est purement illustrative et non limitative et doit être lue en regard des figures annexées, parmi lesquelles :
- la figure 1 représente de manière schématique, en vue de face, un utilisateur équipé d'une structure d'exosquelette,
- la figure 2 représente de manière schématique un sous-ensemble de la structure d'exosquelette conforme à un premier mode de réalisation de l'invention,
- la figure 3 représente de manière schématique un sous-ensemble de la structure d'exosquelette conforme à un deuxième mode de réalisation de l'invention,
- les figures 4A et 4B représentent de manière schématique un premier exemple d'ensemble de liaison lorsque le pion se trouve dans la première position d'extrémité et lorsque le pion se trouve dans la deuxième position d'extrémité respectivement,
- les figures 5A et 5B représentent de manière schématique un deuxième exemple d'ensemble de liaison lorsque le pion se trouve dans la première position d'extrémité et lorsque le pion se trouve dans la deuxième position d'extrémité respectivement,
- la figure 6 représente de manière schématique un troisième exemple d'ensemble de liaison,
- les figures 7A et 7B représentent de manière schématique le troisième exemple d'ensemble de liaison lorsque le pion se trouve dans la première position d'extrémité et lorsque le pion se trouve dans la deuxième position d'extrémité respectivement.

### DESCRIPTION DETAILLEE D'UN MODE DE REALISATION

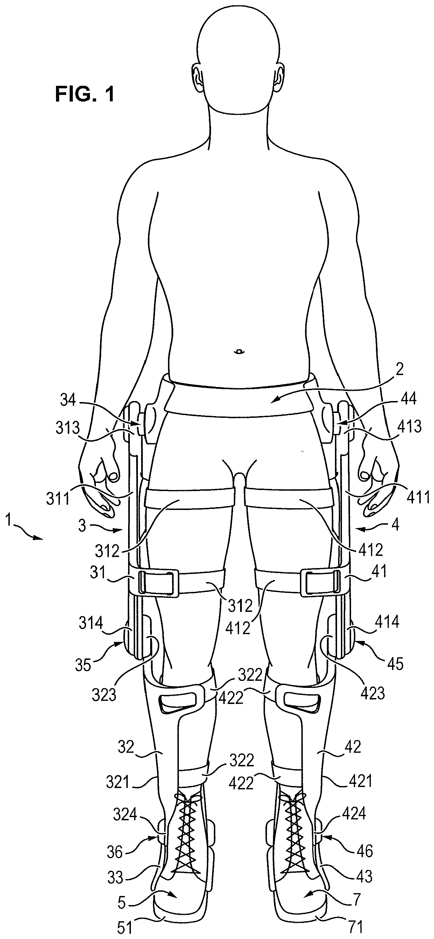

Sur la figure 1, la structure d'exosquelette 1 représentée comprend une ceinture lombaire 2, un premier ensemble mécanique 3 et un deuxième ensemble mécanique 4.

La ceinture lombaire 2 est propre à entourer le bas du tronc de l'utilisateur. Le premier ensemble mécanique 3 est propre à être relié à un premier membre inférieur de l'utilisateur (jambe droite) pour assister le mouvement du premier membre inférieur lors de la marche ou de la course. Le deuxième ensemble mécanique 4 est propre à être relié à un deuxième membre inférieur de l'utilisateur (jambe gauche) pour assister le mouvement du deuxième membre inférieur lors de la marche ou de la course. Le premier ensemble mécanique 3 et le deuxième ensemble mécanique 4 sont raccordés chacun à la ceinture lombaire 2.

Le premier ensemble mécanique 3 comprend une première pièce fémorale 31, une première pièce tibiale 32, et une première pièce de pied 33.

La première pièce fémorale 31 comprend un premier segment fémoral 311 prévu pour s'étendre le long d'une première cuisse (cuisse droite) de l'utilisateur et des sangles de fixation 312 propres à entourer la première cuisse de l'utilisateur pour fixer le segment fémoral 311 à la première cuisse.

La première pièce tibiale 32 comprend un premier segment tibial 321 prévu pour s'étendre le long d'un premier mollet (mollet droit) de l'utilisateur et des sangles de fixation 322 propres à entourer le premier mollet de l'utilisateur pour fixer le segment tibial 321 au premier mollet.

La première pièce de pied 33 est fixée à une première chaussure 5 de l'utilisateur, par exemple à une semelle 51 de la chaussure 5. La première pièce de pied 33 peut être fixée à la semelle 51 au moyen de vis.

Le premier segment fémoral 311 comprend une première extrémité 313 raccordée à la ceinture lombaire 2 par le biais d'une première articulation de hanche 34 et une deuxième extrémité 314 raccordée au premier segment tibial 321 par le biais d'une première articulation de genou 35.

Le premier segment tibial 321 comprend une première extrémité 323 raccordée au premier segment fémoral 311 par la première articulation de genou 35 et une deuxième extrémité 324 raccordée à la première pièce de pied 33 par le biais d'une première articulation de cheville 36.

Le deuxième ensemble mécanique 4 est symétrique du premier ensemble mécanique 3.

Le deuxième ensemble mécanique 4 comprend également une deuxième pièce fémorale 41, une deuxième pièce tibiale 42 et une deuxième pièce de pied 43.

La deuxième pièce fémorale 41 comprend un deuxième segment fémoral 411 prévu pour s'étendre le long d'une deuxième cuisse (cuisse gauche) de l'utilisateur et des sangles de fixation 412 propres à entourer la deuxième cuisse de l'utilisateur pour fixer le segment fémoral 411 à la deuxième cuisse.

La deuxième pièce tibiale 42 comprend un deuxième segment tibial 421 prévu pour s'étendre le long d'un deuxième mollet (mollet gauche) de l'utilisateur et des sangles de fixation 422 propres à entourer le deuxième mollet de l'utilisateur pour fixer le segment tibial 421 au deuxième mollet.

La deuxième pièce de pied 43 est fixée à une deuxième chaussure 7 de l'utilisateur, par exemple à une semelle 71 de la chaussure 7. La deuxième pièce de pied 43 peut être fixée à la semelle 71 au moyen de vis.

Le deuxième segment fémoral 411 comprend une première extrémité 413 raccordée à la ceinture lombaire 2 par le biais d'une deuxième articulation de hanche 44 et une deuxième extrémité 414 raccordée au deuxième segment tibial 421 par le biais d'une deuxième articulation de genou 45.

Le deuxième segment tibial 421 comprend une première extrémité 423 raccordée au deuxième segment fémoral 411 par la deuxième articulation de genou 45 et une deuxième extrémité 424 raccordée à la deuxième pièce de pied 43 par le biais d'une deuxième articulation de cheville 46.

Les articulations de hanche 34, 44 et les articulations de genou 35, 45 peuvent comprendre des actionneurs permettant d'assister l'utilisateur lors d'un mouvement de flexion ou d'extension de la hanche ou du genou.

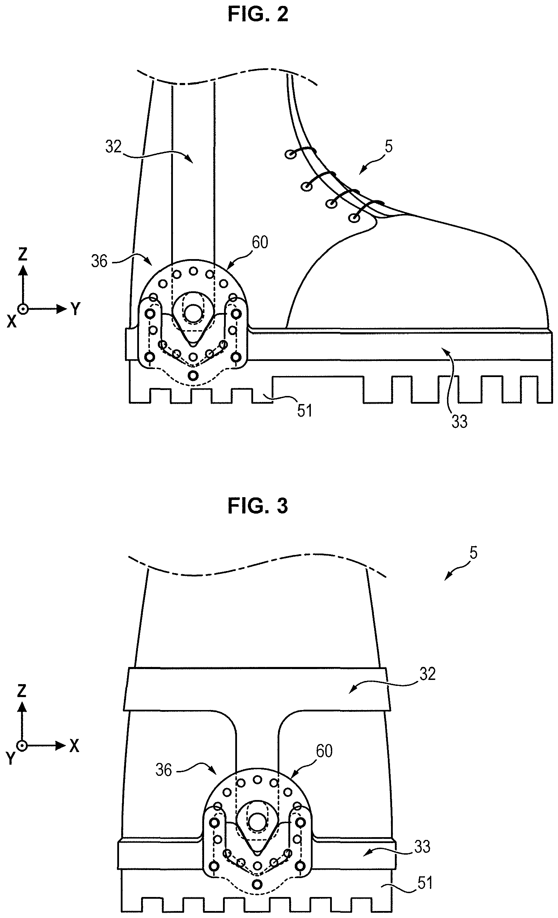

La figure 2 représente plus en détails une articulation de cheville 36 conforme à un premier mode de réalisation de l'invention.

Dans ce premier mode de réalisation, l'articulation de cheville 36 est prévue pour autoriser un mouvement de flexion/extension du pied par rapport à la jambe de l'utilisateur.

Autrement dit, l'articulation de cheville 36 autorise une rotation de la pièce tibiale 32 par rapport à la pièce de pied 33 autour d'un axe de rotation X, parallèle à un axe de flexion/extension de la cheville, lorsque la pièce tibiale 32 est fixée à la jambe et que la pièce de pied 33 est fixée au pied de l'utilisateur.

La figure 3 représente plus en détails une articulation de cheville 36 conforme à un deuxième mode de réalisation de l'invention.

Dans ce deuxième mode de réalisation, l'articulation de cheville 36 est prévue pour autoriser un mouvement d'éversion/inversion du pied de l'utilisateur par rapport à la jambe.

Autrement dit, l'articulation de cheville 36 autorise une rotation de la pièce tibiale 32 par rapport à la pièce de pied 33 autour d'un axe de rotation Y, parallèle à un axe d'éversion/inversion de la cheville lorsque la pièce tibiale 32 est fixée à la jambe et que la pièce de pied 33 est fixée au pied de l'utilisateur.

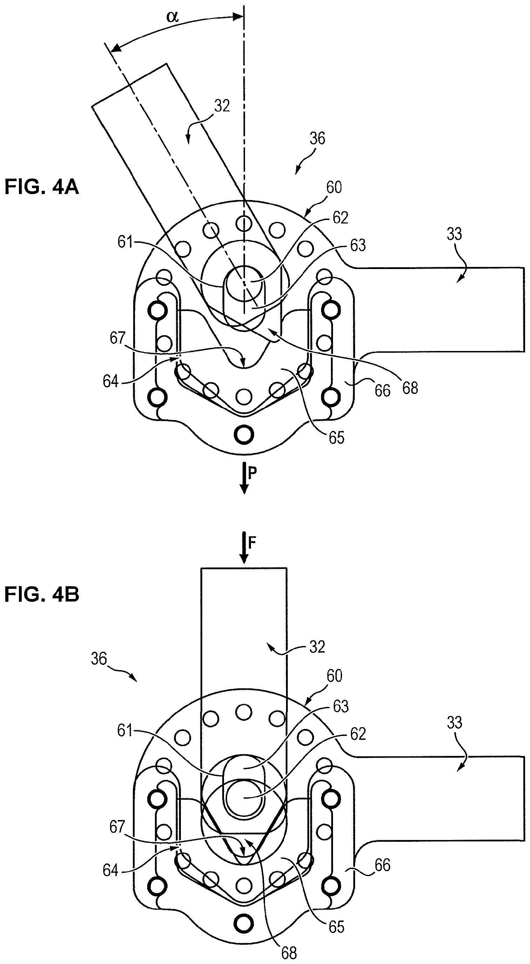

Les figures 4A et 4B illustrent plus en détails la première articulation de cheville 36 conformément à un premier exemple de réalisation. Il est à noter que la deuxième articulation de cheville 46 est identique à la première articulation de cheville 36.

L'articulation de cheville 36 comprend un ensemble de liaison 60 reliant la pièce tibiale 32 à une pièce de pied 33.

L'ensemble de liaison 60 comprend un guide 61 monté fixe par rapport à la pièce tibiale 32, et un pion 62 monté fixe par rapport à la pièce de pied 33. Le pion 62 est monté coulissant à l'intérieur du guide 61 entre une première position d'extrémité (illustrée sur la figure 4A) et une deuxième position d'extrémité (illustrée sur la figure 4B).

Le guide 61 comprend une lumière oblongue 63 ménagée dans la pièce tibiale 32. Le pion 62 s'étend à travers la lumière oblongue 63. Le pion 62 présente une forme cylindrique de révolution, ayant un axe de révolution. De cette manière, le pion 62 peut à la fois être déplacé en translation par rapport au guide 61, et pivoter par rapport au guide 61 selon un axe de rotation X (égal à l'axe de révolution du pion) et perpendiculaire à la direction Z de translation du pion 62 par rapport au guide 61. La rotation t la translation du pion 62 par rapport au guide 61 sont indépendantes.

L'axe de rotation X est un axe de rotation parallèle à l'axe de flexion/extension de la cheville conformément au premier mode de réalisation illustré sur la figure 2.

Toutefois, l'axe de rotation pourrait également être l'axe de rotation Y, parallèle à l'axe d'éversion/inversion de la cheville conformément au deuxième mode de réalisation illustré sur la figure 3.

L'ensemble de liaison 60 comprend en outre un dispositif de limitation 64 agencé pour autoriser une rotation du pion 62 par rapport au guide 61 lorsque le pion 62 est dans la première position d'extrémité (figure 4A), et limiter la rotation du pion 62 par rapport au guide 61 lorsque le pion 62 est dans la deuxième position d'extrémité (figure 4B).

Le dispositif de limitation 64 comprend un élément élastique 65 monté fixe sur la pièce de pied 33. L'élément élastique 65 est monté fixe sur la pièce de pied 33 par exemple au moyen de plaques 66 disposées de part et d'autre de l'élément élastique 65 et visées sur la pièce de pied 33. L'élément élastique 65 est maintenu serré entre les deux plaques 66.

L'élément élastique 65 est par exemple un bloc en matériau élastique, tel que du caoutchouc.

L'élément élastique 65 comprend un renfoncement 67 présentant une forme générale en V. Le renfoncement 67 présente un angle d'ouverture compris entre 20 et 150 degrés, de préférence entre 30 et 40 degrés.

Le dispositif de limitation 60 comprend en outre une saillie 68 montée fixe sur la pièce tibiale 32. La saillie 68 peut être montée fixe sur la pièce tibiale 32 par le biais du pion 62.

Dans le premier exemple illustré sur les figures 4A et 4B, la saillie 68 présente une forme complémentaire de la forme du renfoncement 67. Plus précisément, la saillie 68 présente une forme générale de pointe.

La saillie 68 est propre à être mise en prise avec l'élément élastique 67 lorsque le pion 62 est dans la deuxième position d'extrémité (figure 4B).

Plus précisément, lorsque le pion 62 se trouve dans la deuxième position d'extrémité (figure 4B), la saillie 68 est reçue dans le renfoncement 67 de l'élément élastique 65, ce qui a pour effet de limiter la rotation du pion 62 par rapport au guide 61.

Lorsque l'utilisateur marche, le fonctionnement de l'articulation de cheville 36 est le suivant.

Pendant le cycle de marche, le pied de l'utilisateur passe successivement d'une phase d'appui (c'est-à-dire une phase durant laquelle le pied de l'utilisateur est en appui sur le sol) à une phase d'oscillation (c'est-à-dire une phase durant laquelle le pied de l'utilisateur n'est plus en contact avec le sol).

Pendant la phase d'appui, la charge s'exerçant sur l'exosquelette génère sur l'ensemble mécanique 3 une force F qui a pour effet de solliciter la pièce tibiale 32 vers le bas, et par conséquent de solliciter le pion 62 de l'articulation de cheville 36 vers la deuxième position d'extrémité (figure 4B).

Dans cette deuxième position d'extrémité, la rotation du pion 62 par rapport au guide 61 est limitée. En effet, la saillie 68 est en prise avec l'élément élastique 65. L'élément élastique 65 exerce alors sur la pièce tibiale 32 une force de rappel élastique s'opposant à une rotation relative entre la pièce tibiale 32 et la pièce de pied 33, aussi bien dans un premier sens de rotation, que dans un deuxième sens de rotation opposé au premier sens de rotation. En limitant le mouvement de la saillie 68, l'élément élastique 65 limite le débattement en rotation de la pièce tibiale 32 par rapport à la pièce de pied 33.

Dans cette position, la charge qui s'exerce sur l'exosquelette est transférée de la pièce tibiale 32 à la pièce de pied 33. Cette charge est transférée de la pièce de pied 33 à la chaussure 5 et donc au sol.

Pendant la phase d'oscillation, la charge s'exerçant sur l'exosquelette est transférée principalement vers le sol via l'autre ensemble mécanique 4. En outre, la chaussure 5 n'est plus en contact avec le sol et le poids P de la chaussure 5 sollicite la pièce de pied 33 vers le bas. Le poids P sollicite par conséquent le pion 62 de l'articulation de cheville 46 vers la première position d'extrémité (figure 4A).

Dans cette première position d'extrémité, la saillie 68 n'est plus en prise avec l'élément élastique 65. L'élément élastique 65 ne limite donc plus le débattement en rotation de la pièce tibiale 32 par rapport à la pièce de pied 33. Le dispositif de limitation 60 autorise une rotation de la pièce de pied 33 par rapport à la pièce tibial 32, permettant ainsi une liberté de mouvement de l'utilisateur.

Dans cette première position, aucune charge n'est transférée de la pièce tibiale 32 à la pièce de pied 33.

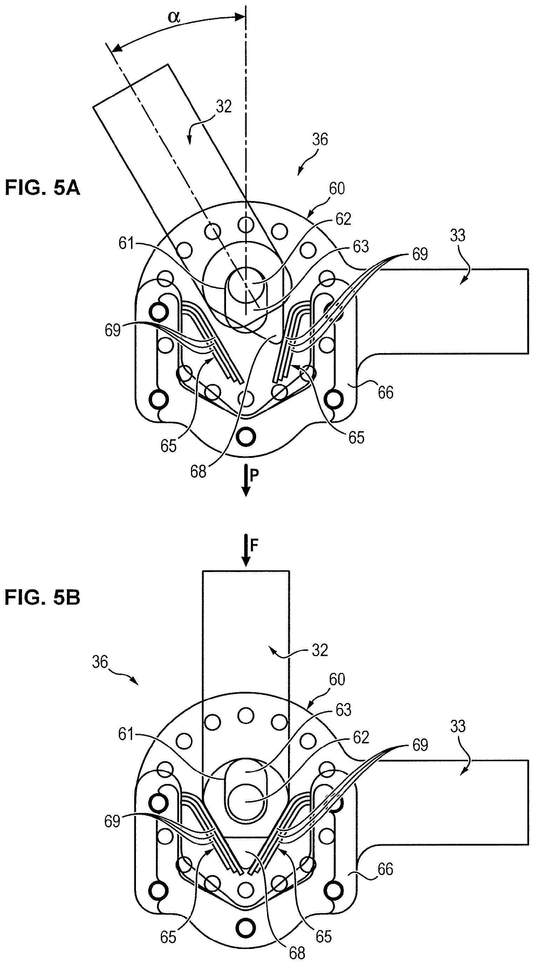

Les figures 5A et 5B illustrent plus en détails la première articulation de cheville 36 conformément à un deuxième exemple de réalisation.

Dans ce deuxième exemple, le dispositif de limitation 64 comprend deux éléments élastiques 65 montés fixe sur la pièce de pied 33. Chaque élément élastique est un ressort à lames.

Les ressorts à lames sont disposés de part et d'autre de la saillie 68 en formant un V.

Chaque ressort à lames 65 comprend une pluralité de lames flexibles 69 agencées parallèlement les unes aux autres. Les lames peuvent être formées en métal, tel qu'en acier par exemple.

Chaque lame 69 présente une première extrémité fixée à la pièce de pied 33 et une deuxième extrémité libre. Les lames flexibles 69 présentent des longueurs différentes de manière à procurer au ressort une flexibilité étagée. Les lames 69 d'un même ressort 65 sont agencées côte à côte de la plus grande à la plus petite, de sorte que lorsque le pion 62 est dans la deuxième position d'extrémité (figure 5B), la saillie 68 entre en contact les lames de plus grande longueur.

Plus précisément, lorsque le pion 62 se trouve dans la deuxième position d'extrémité (figure 5B), la saillie 68 est reçue entre les deux éléments élastiques 65, ce qui a pour effet de solliciter les lames 69 en flexion.

Lorsqu'elles sont sollicitées en flexion, les lames 69 exercent sur la saillie 68 une force de rappel élastique tendant à s'opposer à une rotation du pion 62 par rapport au guide 61.

Lorsque l'utilisateur marche, le fonctionnement de l'articulation de cheville 36 est le suivant.

Pendant la phase d'appui, la charge s'exerçant sur l'exosquelette génère sur l'ensemble mécanique 3 une force F qui a pour effet de solliciter la pièce tibiale 32 vers le bas, et par conséquent de solliciter le pion 62 de l'articulation de cheville 36 vers la deuxième position d'extrémité (figure 5B).

Dans cette deuxième position d'extrémité, la rotation du pion 62 par rapport au guide 61 est possible mais elle est limitée. En effet, la saillie 68 est en contact avec les deux éléments élastiques 65. En s'opposant au mouvement de la saillie 68, les éléments élastiques 65 limitent le débattement en rotation de la pièce tibiale 32 par rapport à la pièce de pied 33.

Dans cette position, la charge qui s'exerce sur l'exosquelette est transférée de la pièce tibiale 32 à la pièce de pied 33. Cette charge est transférée de la pièce de pied 33 à la chaussure 5 et donc au sol.

Pendant la phase d'oscillation, la charge s'exerçant sur l'exosquelette est transférée principalement vers le sol via l'autre ensemble mécanique 4. En outre, la chaussure 5 n'est plus en contact avec le sol et le poids P de la chaussure 5 sollicite la pièce de pied 33 vers le bas. Le poids P sollicite par conséquent le pion 62 de l'articulation de cheville 46 vers la première position d'extrémité (figure 5A).

Dans cette première position d'extrémité, la saillie 68 n'est plus en contact avec les éléments élastiques 65. Les éléments élastiques 65 ne s'opposent donc plus à une rotation de la pièce tibiale 32 par rapport à la pièce de pied 33. Le dispositif de limitation 60 autorise une rotation de la pièce de pied 33 par rapport à la pièce tibial 32, permettant ainsi une liberté de mouvement de l'utilisateur.

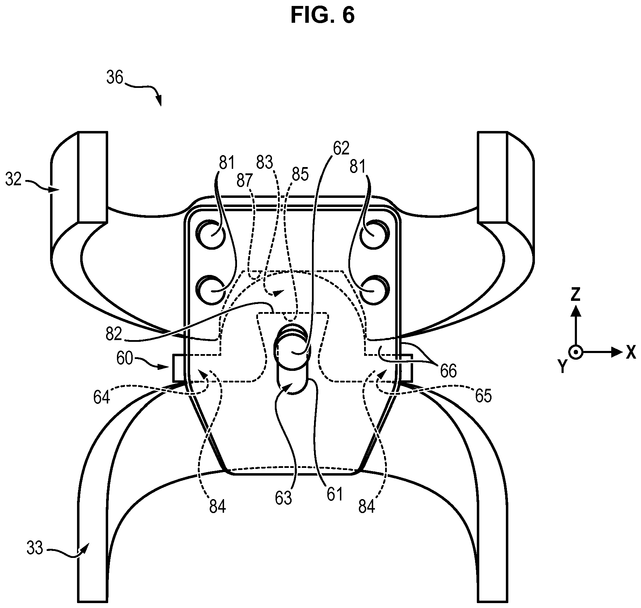

La figure 6 illustre la première articulation de cheville 36 conformément à un troisième exemple de réalisation. Il est à noter que la deuxième articulation de cheville 46 est identique à la première articulation de cheville 36.

L'articulation de cheville 36 comprend un ensemble de liaison 60 reliant la pièce tibiale 32 à la pièce de pied 33.

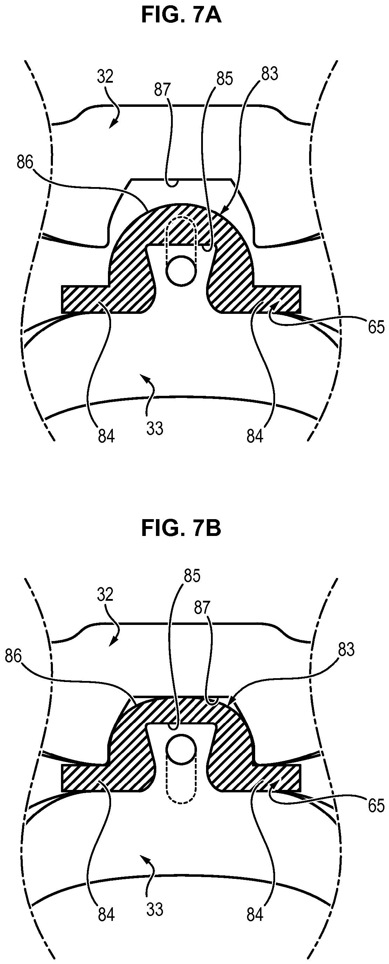

L'ensemble de liaison 60 comprend un guide 61 monté fixe par rapport à la pièce tibiale 32, et un pion 62 monté fixe par rapport à la pièce de pied 33. Le pion 62 est monté coulissant à l'intérieur du guide 61 entre une première position d'extrémité (illustrée sur la figure 7A) et une deuxième position d'extrémité (illustrée sur la figure 7B).

A cet effet, l'ensemble de liaison 60 comprend deux plaques 66, disposées de part et d'autre de la pièce tibiale 32. Les deux plaques 66 sont fixées à la pièce tibiale 32 par le biais de vis de fixation 81 traversant les plaques 66 et la pièce tibiale 32.

Le guide 61 comprend une lumière oblongue 63 ménagée dans l'une des plaques 66 ou de préférence dans les deux plaques 66.

Le pion 62 est fixé à une languette 82 de la pièce de pied 33 s'étendant entre les deux plaques 66.

Le pion 62 s'étend à travers la lumière oblongue 63. Le pion 62 présente une forme cylindrique de révolution, ayant un axe de révolution. De cette manière, le pion 62 peut à la fois être déplacé en translation par rapport au guide 61, et pivoter par rapport au guide 61 selon un axe de rotation Y (égal à l'axe de révolution du pion) et perpendiculaire à la direction Z de translation du pion 62 par rapport au guide 61.

L'axe de rotation Y est un axe de rotation parallèle à l'axe d'éversion/inversion de la cheville conformément au deuxième mode de réalisation illustré sur la figure 3.

Toutefois, l'axe de rotation pourrait également être l'axe de rotation X, parallèle à l'axe de flexion/extension de la cheville conformément au premier mode de réalisation illustré sur la figure 2.

L'ensemble de liaison 60 comprend un dispositif de limitation 64 agencé pour autoriser une rotation du pion 62 par rapport au guide 61 lorsque le pion 62 est dans la première position d'extrémité (figure 7A), et limiter la rotation du pion 62 par rapport au guide 61 lorsque le pion 62 est dans la deuxième position d'extrémité (figure 7B).

Le dispositif de limitation 64 comprend un élément élastique 65 disposé entre la pièce tibiale 32 et la pièce de pied 33. Dans l'exemple illustré sur la figure 6, l'élément élastique 65 est monté fixe sur la pièce de pied 33. A cet effet, l'élément élastique 65 présente une forme qui épouse la languette 82 de la pièce de pied.

L'élément élastique 65 est maintenu entre la pièce tibiale 32 et la pièce de pied 33 au moyen des plaques 66 disposées de part et d'autre de l'élément élastique 65 et visées sur la pièce tibiale 32. L'élément élastique 65 peut néanmoins coulisser entre les deux plaques 66.

L'élément élastique 65 est par exemple un bloc en matériau élastique, tel que du caoutchouc.

L'élément élastique 65 comprend une portion centrale 83 et deux portions latérales 84. La portion centrale 83 présente une forme générale arquée, tandis que chaque portion latérale 84 présente une forme générale droite, de manière à conférer à l'élément élastique 65 une forme générale en Ω.

La portion centrale 83 de l'élément élastique 65 forme ainsi un renfoncement 85 orienté vers la pièce de pied 33. Le renfoncement 84 reçoit la languette 82 de la pièce de pied 33.

La portion centrale 83 de l'élément élastique 65 forme en outre un renflement 86 de forme générale arrondie, orienté vers la pièce tibiale 32.

La pièce tibiale 32 comprend en outre un évidement 87 positionné en regard du renflement 86 et propre à recevoir le renflement 86 de l'élément élastique 65. De cette manière, la pièce tibiale 32 est propre à être mise en prise avec l'élément élastique 65, lorsque le renflement 86 de l'élément élastique est reçu dans l'évidement 87 (figure 7B).

Plus précisément, lorsque le pion 62 se trouve dans la deuxième position d'extrémité (figure 7B), le renflement 86 de l'élément élastique 65 est reçu dans le renfoncement 87 de la pièce tibiale 32, ce qui a pour effet de comprimer la portion centrale 83 de l'élément élastique 65 entre la pièce tibiale 32 et la pièce de pied 33 et de limiter la rotation du pion 62 par rapport au guide 61.

Lorsque l'utilisateur marche, le fonctionnement de l'articulation de cheville 36 est le suivant.

Pendant le cycle de marche, le pied de l'utilisateur passe successivement d'une phase d'appui (c'est-à-dire une phase durant laquelle le pied de l'utilisateur est en appui sur le sol) à une phase d'oscillation (c'est-à-dire une phase durant laquelle le pied de l'utilisateur n'est plus en contact avec le sol).

Pendant la phase d'appui, la charge s'exerçant sur l'exosquelette génère sur l'ensemble mécanique 3 une force F qui a pour effet de solliciter la pièce tibiale 32 vers le bas, et par conséquent de solliciter le pion 62 de l'articulation de cheville 36 vers la deuxième position d'extrémité (figure 7B).

Dans cette deuxième position d'extrémité, la rotation du pion 62 par rapport au guide 61 est possible mais elle est limitée. En effet, le renfoncement 87 de la pièce tibiale 32 est en prise avec l'élément élastique 65. L'élément élastique 65 exerce alors sur la pièce tibiale 32 une force de rappel élastique s'opposant à une rotation relative entre la pièce tibiale 32 et la pièce de pied 33, aussi bien dans un premier sens de rotation, que dans un deuxième sens de rotation opposé au premier sens de rotation.

De plus, l'élément élastique 65 est comprimé entre la pièce tibiale 32 et la pièce de pied 33. Dans cette position, la pièce tibiale 32 peut légèrement tourner par rapport à la pièce de pied autour de l'axe Y. Toutefois, les deux portions latérales 84 de l'élément élastique 65 limitent le débattement en rotation de la pièce tibiale par rapport à la pièce de pied. En effet, en tournant, la pièce tibiale 32 vient en contact avec les portions latérales 84, ces portions latérales 84 exerçant une force de rappel sur la pièce tibiale 32 tendant à s'opposer à la rotation de la pièce tibiale 32 par rapport à la pièce de pied 33.

Dans cette deuxième position d'extrémité, la charge qui s'exerce sur l'exosquelette est transférée de la pièce tibiale 32 à la pièce de pied 33. Cette charge est transférée de la pièce de pied 33 à la chaussure 5 et donc au sol.

Pendant la phase d'oscillation, la charge s'exerçant sur l'exosquelette est transférée principalement vers le sol via l'autre ensemble mécanique 4. En outre, la chaussure 5 n'est plus en contact avec le sol et le poids P de la chaussure 5 sollicite la pièce de pied 33 vers le bas. Le poids P sollicite par conséquent le pion 62 de l'articulation de cheville 46 vers la première position d'extrémité (figure 7A).

Dans cette première position d'extrémité, le renfoncement 87 de la pièce tibiale 32 n'est plus en prise avec l'élément élastique 65. L'élément élastique 65 ne limite donc plus le débattement en rotation de la pièce tibiale 32 par rapport à la pièce de pied 33. Le dispositif de limitation 60 autorise une rotation libre de la pièce de pied 33 par rapport à la pièce tibial 32, permettant ainsi une liberté de mouvement de l'utilisateur.

Dans cette première position, aucune charge n'est transférée de la pièce tibiale 32 à la pièce de pied 33.

## Revendications

1. Sous-ensemble d'exosquelette comprenant :
- une première pièce d'exosquelette (32),
- une deuxième pièce d'exosquelette (33),
- un ensemble de liaison (60) reliant la première pièce d'exosquelette (32) à la deuxième pièce d'exosquelette (33), l'ensemble de liaison (60) comprenant un guide (61) monté fixe par rapport à l'une de la première pièce (32) et de la deuxième pièce (33), et un pion (62) monté fixe par rapport à l'autre de la première pièce (32) et de la deuxième pièce (33), le pion (62) étant monté coulissant à l'intérieur du guide (61) entre une première position d'extrémité et une deuxième position d'extrémité,
dans lequel l'ensemble de liaison (60) comprend en outre un dispositif de limitation (64) agencé pour autoriser une rotation du pion (62) par rapport au guide (61) lorsque le pion (62) est dans la première position d'extrémité, et s'opposer à la rotation du pion (62) par rapport au guide (61) lorsque le pion (62) est dans la deuxième position d'extrémité,
le dispositif de limitation (64) comprenant un élément élastique (65) avec lequel la première pièce d'exosquelette (32) vient en prise lorsque le pion (62) est dans la deuxième position d'extrémité,
**caractérisé en ce que** l'élément élastique (65) exerce sur la première pièce d'exosquelette (33) une force de rappel élastique tendant à s'opposer à une rotation relative entre la première pièce d'exosquelette (33) et la deuxième pièce d'exosquelette (32) aussi bien dans un premier sens de rotation que dans un deuxième sens de rotation, opposé au premier sens de rotation.

2. Sous-ensemble d'exosquelette selon la revendication 1, dans lequel le guide (61) comprend une lumière oblongue (63) ménagée dans la première pièce d'exosquelette (32).

3. Sous-ensemble d'exosquelette selon l'une des revendications 1 et 2, dans lequel le pion (62) présente une forme cylindrique de révolution.

4. Sous-ensemble d'exosquelette selon l'une des revendication 1 à 3, dans lequel l'élément élastique (65) est disposé entre les deux pièces d'exosquelette (32, 33).

5. Sous-ensemble d'exosquelette selon l'une des revendications 1 à 4, dans lequel l'élément élastique (65) est monté fixe par rapport à la deuxième pièce d'exosquelette (33).

6. Sous-ensemble d'exosquelette selon l'une des revendications 1 à 5, dans lequel l'élément élastique (65) est un bloc en matériau élastique.

7. Sous-ensemble d'exosquelette selon l'une des revendications 1 à 6, dans lequel la première pièce d'exosquelette (32) présente une saillie (66), et l'élément élastique (65) présente un renfoncement (67) dans lequel la saillie (66) est reçue lorsque le pion (62) est dans la deuxième position d'extrémité.

8. Sous-ensemble d'exosquelette selon la revendication 7, dans lequel la saillie (66) présente une forme complémentaire de la forme du renfoncement (67).

9. Sous-ensemble d'exosquelette selon l'une des revendications 7 et 8, dans lequel la saillie (66) présente une forme générale de pointe et le renfoncement (67) présente une forme générale en V.

10. Sous-ensemble d'exosquelette selon l'une des revendications 1 à 6, dans lequel la première pièce d'exosquelette (32) présente un évidement (87), et l'élément élastique (65) présente un renflement (86) propre à être reçu dans l'évidement (87) lorsque le pion (62) est dans la deuxième position d'extrémité.

11. Sous-ensemble d'exosquelette selon l'une des revendications 1 à 10, dans lequel l'élément élastique (65) présente une ou plusieurs portion(s) (84) propres à être comprimées entre les deux pièces d'exosquelette (32, 33) lorsque le pion (62) est dans la deuxième position d'extrémité, en cas de rotation relative entre la première pièce d'exosquelette (32) et la deuxième pièce d'exosquelette (33).

12. Sous-ensemble d'exosquelette selon l'une des revendications 1 à 6, dans lequel l'élément élastique (65) comprend un ressort agencé pour exercer une force de rappel sur l'autre pièce d'exosquelette (32), la force de rappel exercée par le ressort s'opposant à la rotation du pion (62) par rapport au guide (61) lorsque le pion (62) est dans la deuxième position d'extrémité.

13. Sous-ensemble d'exosquelette selon la revendication 12, dans lequel le ressort comprend une ou plusieurs lame(s) flexible(s) (69), chaque lame (69) ayant une extrémité fixée à l'une des deux pièces d'exosquelette (33), la ou les lame(s) (69) étant disposée(s) de sorte que la rotation du pion (62) par rapport au guide (61) a pour effet que l'autre pièce d'exosquelette (32) sollicite la ou les lame(s) (69) en flexion.

14. Sous-ensemble d'exosquelette selon l'une des revendications 1 à 13, dans lequel l'une de la première pièce d'exosquelette (32) et de la deuxième pièce d'exosquelette (33) est une pièce propre à être fixée à une jambe d'un utilisateur et l'autre de la première pièce d'exosquelette (32) et de la deuxième pièce d'exosquelette (33) est une pièce propre à être fixée à un pied de l'utilisateur, l'ensemble de liaison (60) autorisant une rotation relative entre la deuxième pièce d'exosquelette (33) et la première pièce d'exosquelette (32) causée par un mouvement d'éversion/inversion du pied par rapport à la jambe ou par un mouvement de flexion/extension du pied par rapport à la jambe.

15. Structure d'exosquelette comprenant un sous-ensemble d'exosquelette conforme à l'une des revendications 1 à 14.

## Patentansprüche

1. Exoskelett-Untereinheit, umfassend:
- ein erstes Exoskelett-Teil (32),
- ein zweites Exoskelett-Teil (33),
- eine Verbindungseinheit (60), die das erste Exoskelett-Teil (32) mit dem zweiten Exoskelett-Teil (33) verbindet, wobei die Verbindungseinheit (60) eine Führung (61) umfasst, die in Bezug auf das eine von dem ersten Teil (32) und dem zweiten Teil (33) fest angebracht ist, und einen Stift (62), der in Bezug auf das andere von dem ersten Teil (32) und dem zweiten Teil (33) fest angebracht ist, wobei der Stift (62) im Inneren der Führung (61) zwischen einer ersten Endposition und einer zweiten Endposition gleitend angebracht ist,
wobei die Verbindungseinheit (60) ferner eine Begrenzungsvorrichtung (64) umfasst, die ausgebildet ist, um eine Rotation des Stifts (62) in Bezug auf die Führung (61) zu gestatten, wenn der Stift (62) in der ersten Endposition ist, und um der Rotation des Stifts (62) in Bezug auf die Führung (61) entgegenzuwirken, wenn der Stift (62) in der zweiten Endposition ist,
wobei die Begrenzungsvorrichtung (64) ein elastisches Element (65) umfasst, mit dem das erste Exoskelett-Teil (32) in Eingriff kommt, wenn der Stift (62) in der zweiten Endposition ist,
**dadurch gekennzeichnet, dass** das elastische Element (65) auf das erste Exoskelett-Teil (33) eine elastische Rückstellkraft ausübt, die dazu tendiert, einer relativen Rotation zwischen dem ersten Exoskelett-Teil (33) und dem zweiten Exoskelett-Teil (32) sowohl in einer ersten Rotationsrichtung als auch in einer zweiten Rotationsrichtung entgegensetzt zur ersten Rotationsrichtung entgegenzuwirken.

2. Exoskelett-Untereinheit nach Anspruch 1, wobei die Führung (61) ein Langloch (63) umfasst, das in das erste Exoskelett-Teil (32) eingearbeitet ist.

3. Exoskelett-Untereinheit nach einem der Ansprüche 1 und 2, wobei der Stift (62) eine zylindrische Drehform aufweist.

4. Exoskelett-Untereinheit nach einem der Ansprüche 1 bis 3, wobei das elastische Element (65) zwischen den zwei Exoskelett-Teilen (32, 33) angeordnet ist.

5. Exoskelett-Untereinheit nach einem der Ansprüche 1 bis 4, wobei das elastische Element (65) in Bezug auf das zweite Exoskelett-Teil (33) fest angebracht ist.

6. Exoskelett-Untereinheit nach einem der Ansprüche 1 bis 5, wobei das elastische Element (65) ein Block aus elastischem Material ist.

7. Exoskelett-Untereinheit nach einem der Ansprüche 1 bis 6, wobei das erste Exoskelett-Teil (32) einen Vorsprung (66) aufweist und das elastische Element (65) eine Verstärkung (67) aufweist, in der der Vorsprung (66) aufgenommen ist, wenn der Stift (62) in der zweiten Endposition ist.

8. Exoskelett-Untereinheit nach Anspruch 7, wobei der Vorsprung (66) eine komplementäre Form zu der Form der Verstärkung (67) aufweist.

9. Exoskelett-Untereinheit nach einem der Ansprüche 7 und 8, wobei der Vorsprung (66) eine allgemeine Spitzenform aufweist und die Verstärkung (67) eine allgemeine V-Form aufweist.

10. Exoskelett-Untereinheit nach einem der Ansprüche 1 bis 6, wobei das erste Exoskelett-Teil (32) eine Aussparung (87) aufweist und das elastische Element (65) eine Verdickung (86) aufweist, die imstande ist, in der der Aussparung (87) aufgenommen zu sein, wenn der Stift (62) in der zweiten Endposition ist.

11. Exoskelett-Untereinheit nach einem der Ansprüche 1 bis 10, wobei das elastische Element (65) einen oder mehrere Abschnitte (84) aufweist, die imstande sind, zwischen den zwei Exoskelett-Teilen (32, 33) komprimiert zu sein, wenn der Stift (62) in der zweiten Endposition ist, im Fall relativer Rotation zwischen dem ersten Exoskelett-Teil (32) und dem zweiten Exoskelett-Teil (33).

12. Exoskelett-Untereinheit nach einem der Ansprüche 1 bis 6, wobei das elastische Element (65) eine Feder umfasst, die ausgebildet ist, um eine Rückstellkraft auf das andere Exoskelett-Teil (32) auszuüben, wobei die von der Feder ausgeübte Rückstellkraft der Rotation des Stifts (62) in Bezug auf die Führung (61) entgegenwirkt, wenn der Stift (62) in der zweiten Endposition ist.

13. Exoskelett-Untereinheit nach Anspruch 12, wobei die Feder eine oder mehrere flexible Lamellen (69) umfasst, wobei jede Lamelle (69) ein Ende hat, das an einem der zwei Exoskelett-Teile (33) befestigt ist, wobei die Lamelle(n) (69) derart angeordnet ist/sind, dass die Rotation des Stifts (62) in Bezug auf die Führung (61) bewirkt, dass das andere Exoskelett-Teil (32) die Lamelle(n) (69) biegt.

14. Exoskelett-Untereinheit nach einem der Ansprüche 1 bis 13, wobei das eine von dem ersten Exoskelett-Teil (32) und von dem zweiten Exoskelett-Teil (33) ein Teil ist, das imstande ist, an einem Bein eines Benutzers befestigt zu sein und das andere von dem ersten Exoskelett-Teil (32) und dem zweiten Exoskelett-Teil (33) ein Teil ist, das imstande ist, an einem Fuß des Benutzers befestigt zu sein, wobei die Verbindungseinheit (60) eine relative Rotation zwischen dem zweiten Exoskelett-Teil (33) und dem ersten Exoskelett-Teil (32) gestattet, die von einer Eversions-/Inversionsbewegung des Fußes in Bezug auf das Bein oder von einer Flexions-/Extensionsbewegung des Fußes in Bezug auf das Bein verursacht wird.

15. Exoskelett-Struktur, umfassend eine Exoskelett-Untereinheit nach einem der Ansprüche 1 bis 14.

## Claims

1. An exoskeleton subassembly comprising:
- a first exoskeleton part (32),
- a second exoskeleton part (33),
- a connecting assembly (60) connecting the first exoskeleton part (32) to the second exoskeleton part (33), the connecting assembly (60) comprising a guide (61) fixedly mounted with respect to one of the first part (32) and of the second part (33), and a pin (62) fixedly mounted with respect to the other of the first part (32) and of the second part (33), the pin (62) being slidably mounted inside the guide (61) between a first end position and a second end position,
wherein the connecting assembly (60) further comprises a limiting device (64) arranged to allow rotation of the pin (62) with respect to the guide (61) when the pin (62) is in the first end position, and to oppose rotation of the pin (62) with respect to the guide (61) when the pin (62) is in the second end position,
the limiting device (64) comprising an elastic element (65) with which the first exoskeleton part (32) engages when the pin (62) is in the second end position,
**characterized in that** the elastic element (65) exerts on the first exoskeleton part (33) an elastic return force tending to oppose relative rotation between the first exoskeleton part (33) and the second exoskeleton part (32) both in a first direction of rotation and in a second direction of rotation, opposite to the first direction of rotation.

2. The exoskeleton subassembly according to claim 1, wherein the guide (61) comprises an oblong orifice (63) provided in the first exoskeleton part (32).

3. The exoskeleton subassembly according to one of claims 1 and 2, wherein the pin (62) has an axially symmetrical shape.

4. The exoskeleton subassembly according to one of claims 1 to 3, wherein the elastic element (65) is disposed between the two exoskeleton parts (32, 33).

5. The exoskeleton subassembly according to one of claims 1 to 4, wherein the elastic element (65) is fixedly mounted with respect to the second exoskeleton part (33).

6. The exoskeleton subassembly according to one of claims 1 to 5, wherein the elastic element (65) is a block made of elastic material.

7. The exoskeleton subassembly according to one of claims 1 to 6, wherein the first exoskeleton part (32) has a protrusion (66), and the elastic element (65) has a recess (67) in which the protrusion (66) is received when the pin (62) is in the second end position.

8. The exoskeleton subassembly according to claim 7, wherein the protrusion (66) has a shape complementary to the shape of the recess (67).

9. The exoskeleton subassembly according to one of claims 7 and 8, wherein the protrusion (66) has a general shape of a point and the recess (67) has a general V shape.

10. The exoskeleton subassembly according to one of claims 1 to 6, wherein the first exoskeleton part (32) has a cutout (87), and the elastic element (65) has a bulge (86) capable of being received in the cutout (87) when the pin (62) is in the second end position.

11. The exoskeleton subassembly according to one of claims 1 to 10, wherein the elastic element (65) has one or more portion(s) (84) capable of being compressed between the two exoskeleton part (32, 33) when the pin (62) is in the second end position, in the event of relative rotation between the first exoskeleton part (32) and the second exoskeleton part (33).

12. The exoskeleton subassembly according to one of claims 1 to 6, wherein the elastic element (65) comprises a spring arranged to exert a return force on the other exoskeleton part (32), the return force exerted by the spring opposing to the rotation of the pin (62) with respect to the guide (61) when the pin (62) is in the second end position.

13. The exoskeleton subassembly according to claim 12, wherein the spring comprises one or more flexible blade(s) (69), each blade (69) having one end attached to one of the two exoskeleton part (33), the blade(s) (69) being disposed so that the rotation of the pin (62) with respect to the guide (61) has the effect that the other exoskeleton part (32) loads the blade(s) (69) in flexure.

14. The exoskeleton subassembly according to one of claims 1 to 13, wherein one of the first exoskeleton part (32) and of the second exoskeleton part (33) is a part capable of being attached to a leg of the user and the other of the first exoskeleton part (32) and of the second exoskeleton part (33) is a part capable of being attached to a foot of the user, the connecting assembly (60) allowing relative rotation between the second exoskeleton part (33) and the first exoskeleton part (32) caused by an eversion/inversion movement of the foot with respect to the leg or by a flexural/extension movement of the foot with respect to the leg.

15. An exoskeleton structure comprising an exoskeleton subassembly according to one of claims 1 to 14.
